# EUROPEAN PATENT SPECIFICATION

(11) **EP 2 556 043 B1**
(45) Date of publication and mention of the grant of the patent: **26.10.2016**
(21) Application number: 11720872.8
(22) Date of filing: 07.04.2011
(51) Int. Cl.: C07C 69/587, C07C 233/45, C07K 4/00, A61K 8/37, A61K 8/44, A61K 8/64, A61K 31/22, A61K 31/16, A61K 38/03, A61P 17/00, A61Q 19/00

(54) **NEW POLYTERPENE TYPE COMPOUNDS, COMPOSITIONS CONTAINING THEM AND TOPICAL USES THEREOF**
NEUE VERBINDUNGEN VOM POLYTERPENTYP, ZUSAMMENSETZUNGEN DAMIT UND TOPISCHE VERWENDUNGEN DAVON
NOUVEAUX COMPOSÉS DE POLYTERPÈNE, COMPOSITIONS LES RENFERMANT ET UTILISATIONS TOPIQUES

(30) Priority: 08.04.2010 FR 1052672
(43) Date of publication of application: 13.02.2013
(73) Proprietor: SEDERMA, 78610 Le Perray en Yvelines (FR)
(72) Inventor: FOURNIAL, Arnaud, F-75016 Paris (FR); MONDON, Philippe, F-92120 Montrouge (FR); PESCHARD, Olivier, F-28300 Saint Prest (FR)
(86) International application number: PCT/IB2011/051500
(87) International publication number: WO 2011/125040

(56) References cited:
- JP-A- 2008 189 607
- US-A- 5 756 109
- US-A1- 2006 269 496
- US-B1- 6 187 814
- DATABASE CA [Online] CHEMICAL ABSTRACTS SERVICE, COLUMBUS, OHIO, US; VLAD, P. F. ET AL: "Cyclization of high terpenoid acids and their esters in superacid", XP002607880, retrieved from STN Database accession no. 1996:188582 & VLAD, P. F. ET AL: "Cyclization of high terpenoid acids and their esters in superacid", IZVESTIYA AKADEMII NAUK, SERIYA KHIMICHESKAYA , (12), 2507-13 CODEN: IASKEA, 1995,
- DATABASE CA [Online] CHEMICAL ABSTRACTS SERVICE, COLUMBUS, OHIO, US; MURAKAMI, MANABU ET AL: "Effect of synthetic acyclic polyisoprenoids on the cold-restraint stress induced gastric ulcer in rats", XP002607881, retrieved from STN Database accession no. 1983:447553 & MURAKAMI, MANABU ET AL: "Effect of synthetic acyclic polyisoprenoids on the cold-restraint stress induced gastric ulcer in rats", JAPANESE JOURNAL OF PHARMACOLOGY , 33(3), 549-56 CODEN: JJPAAZ; ISSN: 0021-5198, 1983,

## Description

### TECHNICAL FIELD

The present invention relates to novel compounds of terpenic type, in particular of geranylgeranyl type (including four terpene patterns, also known as isoprene), compositions comprising them and topical uses thereof.

The present invention concerns the chemical, dermo-medical or cosmetical industries for the hygiene and personal care of the skin and apendages (such as hair, eyelashes, eyebrows, nails, hairs) of mammals, animals or humans.

### BACKGROUND ART

Geranylgeranyl pattern is coupled to many cellular proteins, regulating their activity as well as addressing them in different cellular compartments. The transfer on the proteins is performed by a geranylgeranyltransferase which ensures the coupling with the energy released during the rupture of the phosphate bond of isoprene skeleton-PO4:

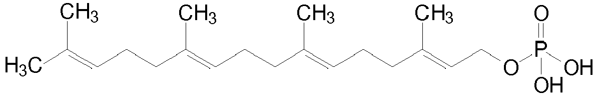

Generally, isoprenes have affinities with the protein helical transmembrane parts and promote by their presence the protein-protein interactions by improving their mobility within the membranes. These protein accompanying properties make the geranylgeranyl isoprene in particular a pivot molecule of the cellular function both at the level of the membrane receptors and the level of the functionality of intracellular proteins and of their addressing. It is a sort of physiological facilitator.

Compounds comprising a pattern of geranylgeranyl type have already been proposed and in cosmetics such as:
- Geranylgeraniol in anti-aging, active promoter of collagen synthesis (JP2008189607) and having the following formula: 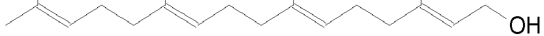 This compound is also described in combination with retinol also for anti-aging properties (US5756109).
- Geranyl geranyl acetone called teprenone known for its whitening (EP0561305), anti-aging (EP1879538) and humectant (JP7309710) properties and having the following formula: 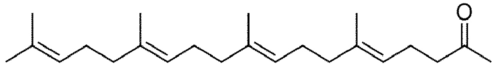 The following documents disclose the use of esters of farnesoate for therapeutical treatments:
- US6187814 discloses the use of esters of farnesoate as activators of the nuclear receptor farnesoid X-activated receptor (FXR) for a therapeutical treatment of subjects suffering from skin or mucous membrane diseases or disorders which display disruptions of the barrier function, and those that involve disorders of the epidermal differentiation and proliferation ; and
- US2006/269496 discloses also the use of esters of farnesoate as agonists of FXR for reducing hair growth.

The article "Cyclisation of high terpenoids acids and their esters in superacid" in the Journal "Izvestiya Zkademii Nauk, Seriya Khimicheskaya", (1995), (12), 2507-13, discloses the terpenoid compound:

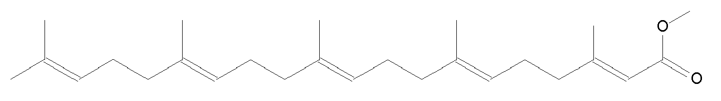

Thereafter in the description called the GGPM (formula V).

The article "Effect of synthetic acyclic polyisoprenoids on the cold-restraint stress induced gastric ulcer in rats" in the Japanese Journal of Pharmacology (1983), 33(3), 549-56, discloses the terpenoid compound and its anti-ulcer activity:

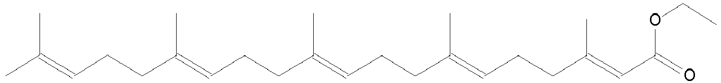

Thereafter in the description called the GGPE (formula IV).

The present invention aims to propose alternative compounds for cosmetic and pharmacy industries still in demand for new ingredients, including ingredients that may be active on several targets in order to offer a range of complementary activities.

### SUMMARY OF THE INVENTION

To this end, the present invention proposes polyterpenic compounds corresponding to formula I below: 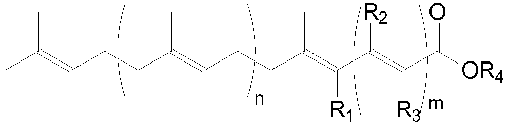 with n =3, m = 0 or 1, R₁, R₂ and R₃, independently of each other a methyl or a hydrogen atom and R₄ is an ethyl or methyl group,
with the exclusion of the compounds corresponding in formula I to n=3, m=0,R₁=H and R₄=methyl or ethyl (corresponding respectively to the GGPM and the GGPE mentioned above, accidentally disclosed in the prior art).

Corresponding amino-acid or peptides derivatives corresponding to the following formula VI 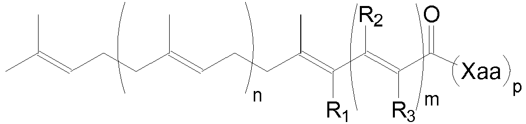 (Xaa)ₚ entity being either an amino-acid (with p=1), or any peptide having p amino-acids with p between 1 and 10, are disclosed herein.
n=3 in formulas I and VI so as to form a geranyl geranyl group.

The present invention also discloses the chemical analogues of these compounds.

As further detailed in the description, results of *in vitro* tests have been obtained with the compounds of the invention that open a strong potential for applications in cosmetics, nutraceutics or pharmaceutics in particular dermatology. "Oleodensifying" properties, an alternative to the "lipofilling", giving volume through the stimulation of adipogenesis and thus allowing compensating subcutaneous volume deficits, depigmenting, anti-oxidant and firming properties were in particular highlighted.

According to other preferred particular features R₂ is preferably a methyl.

The invention encompasses in particular the following compounds:
- The Geranyl Geranyl Ethyl Hexanoate having the following developed formula II and called thereafter in the description the GGHE: 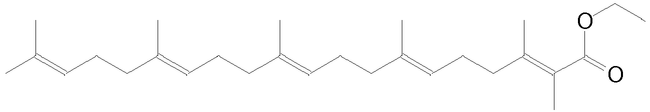 With n = 3, m = 0, R₁ = methyl and R₄ = ethyl.
- The Geranyl Geranyl Ethyl Octadienoate of the following developed formula III and called thereafter in the description the GGOE: 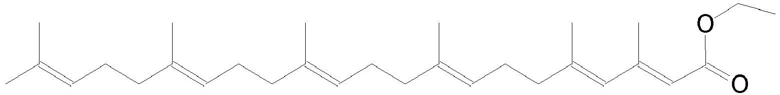 With n = 3, m = 1, R₁ = H, R₂ = methyl, R₃ = H and R₄ = ethyl. Farnesyl type compounds (n=2) are also examples within the scope of the present invention.

The manufacturing process can advantageously be based on a Wittig reaction with teprenone and the adequate triphenylphosphine Wittig reagent as starting reagents, as can be seen in the reaction scheme given below, illustrate the GGOE:

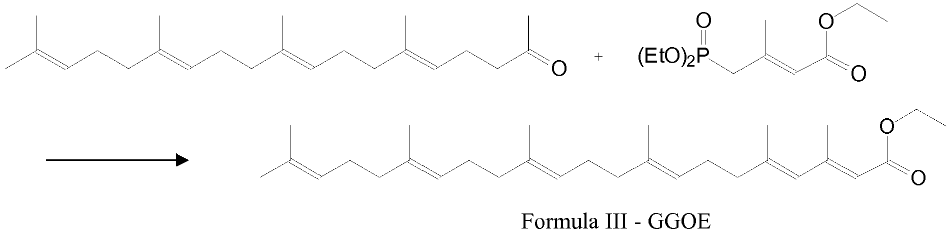

Other manufacture processes are of course possible.

Advantageously, as mentioned above, the invention compounds can be coupled to a (Xaa)p entity which is either an amino-acid (with p=1), or any peptide having p amino-acids with p between 2 and 10 in order to obtain compounds of the following developed formula, according to the following reaction scheme: 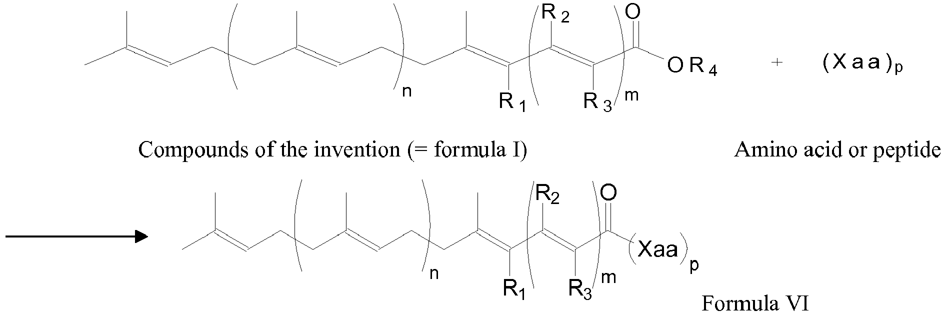 with p comprised between 1 and 10.

The compounds of the invention thus constitute an alternative to conventional lipid chains linked to peptides, as a palmitoyl or elaidoyl chain, whose function is to improve the bioavailability of peptide and its ability to penetrate the skin, and to form a synergy with the coupled peptide activity.

The invention covers peptides consisting of encoded amino acids, natural or unnatural, derivatives and analogs, pure or in mixtures (encoded amino acids: Alanine A Ala, Arginine R Arg, Asparagine N Asn, Aspartate or aspartic acid D Asp, Cysteine C Cys, Glutamate or glutamic acid E Glu, Glutamine Q Gln, Glycine G Gly, Histidine H His, I Ile Isoleucine, Leucine L Leu, Lysine K Lys, Methionine M Met, Phenylalanine F Phe, Proline P Pro, Serine S Ser, Threonine T Thr, Tryptophan W Trp, Tyrosine Y Tyr, Valine V Val). Are cited for example, without limitation, the amino acids K, T, C, M, MO (methionine whose sulfur is oxidized), MO2 (methionine whose sulfur is dioxidized), the dipeptides KT, KC, KP, VW, KK or TT, the tripeptides GHK, GKH, KPK, KMOK, KMO2K or KAvaK, the tetrapeptides GQPR, the pentapeptides KTTKS, the hexapeptides GKTTKS, etc.

As for other examples, the peptides of the following peptide formulations can be coupled: Matrixyl^{®} [INCI: Palmitoyl Pentapeptide-3], Matrixyl^{®} 3000 [INCI: Palmitoyl Tetrapeptide-3, Palmitoyl Oligopeptide], Matrixyl^{®} synthe'6™ [Glycerin, Water (Aqua), Hydroxypropyl Cyclodextrin, Palmitoyl Tripeptide-38], Calmosensine™ [INCI: Acetyl Dipeptide-1 Cetyl Ester], Rigin™ [INCI: Palmitoyl Tetrapeptide -7], Procapil™ [INCI: Apigenin, Oleanolic Acid, Biotinoyl Tripeptide-1], Dermaxyl™ [INCI: Ceramide 2, Palmitoyl Oligopeptide], Volulip™ [INCI: Portulaca Pilosa Extract, Palmitoyl Tripeptide-38], Biopeptide EL™ [INCI: Palmitoyl Oligopeptide], Biopeptide CL™ [INCI: Palmitoyl Oligopeptide], Vexel™ [INCI: Caffeine, Palmitoyl Carnitine] or Bio-Bustyl™ [INCI: Rahnella Soy Protein Ferment, Palmitoyl Oligopeptide] marketed by Sederma; Vialox™ [INCI: Pentapeptide-3], Syn-ake™ [INCI: Dipeptide Diaminobutyroyl Benzylamide Diacetate] or Syn-Coll™ [INCI: Palmitoyl Tripeptide-5] marketed by Pentapharm, Hydroxyprolisilane CN™ [INCI: Methylsilanol Hydroxyproline Aspartate] marketed by Exsymol, Argireline™ [INCI: Acetyl Hexapeptide-8], Leuphasyl™ [INCI: Pentapeptide-18], Aldenine™ [INCI: Hydrolized wheat protein, hydrolized soy protein, tripeptide-1], Trylgen™ [INCI: Pseudoalteromonas Ferment Extract, Hydrolyzed Wheat Protein, Hydrolyzed Soy Protein, Tripeptide-10 Citrulline, Tripeptide-1], Eyeseryl™ [INCI: Acetyl Tetrapeptide-5], Serilesine™ [INCI: hexapeptide-10] or Decorinyl™ [INCI: Tripeptide-10 Citrulline] marketed by Lipotec, Kollaren™ [INCI: Tripeptide-1, Dextran], IP2000™ [INCI: Dextran, Trifluoroacetyl tripeptide-2] or Meliprene™ [INCI: Dextran, Acetyl Heptapeptide-1] marketed by the lnstitut Europeen de Biologie Cellulaire, Collaxyl™ [INCI: Hexapeptide-9] or Quintescine™ [INCI: Water, Butylene Glycol, Dipeptide-4] marketed by Vincience, BONT-L-Peptide™ [proposed INCI: Palmitoyl Hexapeptide] marketed by lnfinitec Activos, Cytokinol™LS [INCI: Hydrolyzed Casein, Hydrolyzed Yeast Protein, Lysine HCL] marketed by the Laboratoires Serobiologiques/Cognis, Neutrazen™ [INCI: Water (Aqua), Butylen Glycol, Dextran, Palmitoyl Tetrapeptide-8] marketed by Innovations, ECM-Protect™ [proposed INCI: Water (Aqua), Dextran, Tripeptide-2] marketed by Atrium Innovations, Timp-Peptide™ [proposed INCI: Acetyl Hexapeptide] or ECM Moduline™ [proposed INCI: Palmitoyltripeptide] marketed by lnfinitec Activos, as well as the peptides further cited in the text.

Beyond ten amino acids, amino acids derivatives or analogs, peptides are generally too bulky for cosmetic applications and too expensive to manufacture. For these reasons, coupled peptide can be limited to n = 6 (hexapeptides).

According to preferred examples, in formula VI:
- n = 3, R₁ = methyl, m = 0 and R₄ = ethyl, corresponding to the coupling of the GGHE with an amino-acid or a peptide.
- n = 3, R₁ = H, m = 1, R₂ = methyl, R₃ = H and R₄ = ethyl, corresponding to the coupling of the GGOE with an amino-acid or a peptide.
- n = 3, R₁ = H, m = 0 and R₄ = ethyl, corresponding to the coupling of the GGPE with an amino-acid or a peptide.
- n = 3, R₁ = H, m = 0 and R₄ = methyl, corresponding to the coupling of the GGPM with an amino-acid or a peptide.

*In-vitro* test results given in the following detailed description show that the compounds of the invention are more particularly useful for preventing or treating the cutaneous signs of aging, in particular through anti-radical and anti-oxydant actions, protection against UV radiations, improvement of the mechanical properties of skin (elasticity, firmness, compressibility), lightening of the skin and treatment of melanin spots (all indication in relation with the decrease of melanogenesis), and for gaining a "lipofilling" effect (restoring/giving volume by stimulating the expansion of adipose tissue).

The present invention also proposes a topical composition, characterized in that it comprises, in a physiologically acceptable medium, as active ingredient, an effective amount of one or more compounds to formula I: 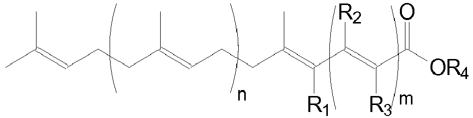 with n =3, m = 0 or 1, R₁, R₂ and R₃, independently of each other a methyl or a hydrogen atom and R₄ is an ethyl or methyl group,

with the exclusion of the compounds corresponding in formula I to n=3, m=0,R₁=H and R₄=methyl or ethyl (corresponding to the GGPM and the GGPE).Such composition can be used in the cosmetical field to improve the overall appearance/condition of the skin and in dermo-pharmaceutical field to for preventing or treating disorders and diseases of the skin and appendages.

According to other preferred particular featuresR₂ is preferably a methyl.

Examples of compounds that can be used for the composition of the invention are:
The GGHE of formula II mentioned above;
The GGOE of formula III mentioned above;
The present invention is more particularly directed to the use of a composition as recited in claim 9 for the cosmetic non therapeutic treatment of skin conditions to improve the overall appearance of the skin, more particularly for the treatments of the signs of intrinsic and extrinsic skin aging, sagging skin, of loss of skin tone, firmness or elasticity, skin atrophy, loss of dermis and epidermis density, for giving volume to the dermis and epidermis, for the treatment of skin dehydration, hair loss or cellulite, for lightening the skin, for the treatment of glycation of molecules in the skin, of acne, of skin breakdown due to the effects of oxidation and to treat inflammatory conditions.

In particular in cosmetics, applications can be offered in the ranges of moisturizers, cleansers, anti-aging, antioxidant, protective, restorative (hands, feet, lips), contours (face, eyes, neck, lips), make-up for skin and appendages, including eyelashes, lip products, solar products, remodeling, plumping, swelling (e.g. of the hands, bottocks, bust, breasts), hair products, etc..

According to other advantageous features, the cosmetic or dermopharmaceutical composition of the invention may incorporate one or more additional active ingredients, to provide advantageously a cosmetic or dermo-pharmaceutical product with a wider range of properties or to enhance the properties of the compounds of the present invention. Additional active ingredients may for example be selected from the lightening, anti-redness, sunscreens, moisturizing, humectants, exfoliating, anti-aging, anti-wrinkle and fine lines, stimulating the collagen and/or elastin synthesis, volumizing, elastic properties improving, anti-acne, anti-inflammatory, anti-oxidants, anti-free radical, depigmenting agents, depilatories, anti-regrowth or promoting the growth agents, peptides, vitamins etc. These active ingredients may be obtained from plant materials such as plant extracts or products from plant cells culture or fermentation.

More specifically, the compound of the invention can be combined with at least one of compounds selected from compounds of vitamin B3, niacinamide compounds like or tocopherol, retinol, hexamidine, α-lipoic acid, resveratrol or DHEA or N-acetyl-Tyr-Arg-O-hexadecyl, Pal-KT, Pal-VGVAPG (SEQ ID NO:1), Pal-KTTKS (SEQ ID NO:2), Pal-GHK, Pal-KMO2K and Pal-GQPR (SEQ ID NO:3) peptides, which are active ingredients used in conventional cosmetic or dermopharmaceutical topical compositions.

### DETAILED DESCRIPTION

The term "physiological medium" means according to the present invention, without limitation, an aqueous or alcoholic solution, a water-in-oil emulsion, an oil-in-water emulsion, a microemulsion, an aqueous gel, an anhydrous gel, a serum, a dispersion of vesicles.

"Physiologically acceptable "means that the disclosed compositions or compounds are suitable for use in contact with mucous membranes, nails, scalp, hairs, hair and skin of mammals and more particularly human without risk of toxicity of incompatibility, instability, allergic response, and others.

When present in a composition, the compound of the invention is present in amounts ranging from 0.000001% to 15% compared to the total weight of the composition, more preferably between 0.0001% and 5%, depending of the destination of the composition and the desired effect more or less pronounced. All percentages and ratios used herein are by weight of total composition and all measurements are made at 25°C unless it is specified otherwise.

Typically, in a composition of the invention consisting simply of the compound of the invention and of an excipient (the physiologically acceptable medium) used as solubilizer, for example, forming an "active ingredient" for the future preparation of a cosmetic composition, the amount of the compound will be comprised between 0.005% and 5%.

The choice of the excipient of the composition is made according to the constraints related to the compounds of the invention (stability, solubility, etc.) and if according to the dosage form then considered for the composition.

The compounds of the invention have solubility in water that varies according to their exact chemical nature. Thus the compounds of the invention can be incorporated into compositions using an aqueous solution, and those that are not soluble in water can be solubilized with cosmetically, pharmaceutically or physiologically acceptable conventional solubilizers, for example and without limiting this list: ethanol, propanol, isopropanol, propylene glycol, glycerin, butylene glycol, or polyethylne glycol or any combination. It may also be interesting to dissolve the compounds of the invention using emulsifiers and for example emulsifiers containing phosphorus such as phosphate esters.

### Additional ingredients

The CTFA International cosmetic ingredient dictionary & handbook (13th Ed. 2010) (published by the Cosmetic, Toiletry, and Fragrance Association, Inc., Washington, D.C.) describes a non limited wide variety of cosmetic and pharmaceutical ingredients usually used in the skin care industry that can be used as additional ingredients in the compositions of the present invention. Examples of these ingredient classes include, but are not limited to: healing agents, skin anti-aging agents, anti-wrinkle agents, anti-atrophy agents, skin moisturizing agents, skin smoothing agents, antibacterial agents, pesticides anti parasitic agents, antifungal agents, fungicidal agents, fungistatic agents, bactericidal agents, bacteriostatic agents, antimicrobial agents, anti-inflammatory agents, anti-pruriginous agents, external anesthetic agents, antiviral agents, keratolytic agents, free radicals scavengers, antiseborrheic agents, antidandruff agents, the agents modulating the differentiation, proliferation or pigmentation of the skin and agents accelerating penetration, desquamating agents, melanin synthesis stimulating or inhibiting agents, whitening or depigmenting agents, propigmenting agents, self-tanning agents, NO-synthase inhibiting agents, antioxidants, free radical scavengers and/or agents against atmospheric pollution, reactive carbonyl species scavengers, antiglycation agents, tightening agents, agents stimulating the synthesis of dermal or epidermal macromolecules and/or capable of inhibiting or preventing their degradation, such as for example collagen synthesis-stimulating agents, elastin synthesis-stimulating agents, decorin synthesis-stimulating agents, laminin synthesis-stimulating agents, defending synthesis-stimulating agents, chaperone synthesis-stimulating agents, aquaporin synthesis stimulation agents, hyaluronic acid synthesis-stimulating agents, fibronectin synthesis stimulating agents, sirtuin synthesis-stimulating agents, agents stimulating the synthesis of lipids and components of the stratum corneum (ceramides, fatty acids, etc.), agents that inhibit collagen degradation, other agents that inhibit elastin degradation, agents that inhibit serine proteases such cathepsin G, agents stimulating fibroblast proliferation, agents stimulating keratinocyte proliferation, agents stimulating adipocyte proliferation, agents stimulating melanocyte proliferation, agents stimulating keratinocyte differentiation, agents stimulating adipocyte differentiation, agents that inhibit acetylcholinesterase, skin relaxant agents, glycosaminoglycan synthesis-stimulating agents, antihyperkeratosis agents, comedolytic agents, antipsoriasis agents, DNA repair agents, DNA protecting agents, stabilizers, anti-itching agents, agents for the treatment and/or care of sensitive skin, firming agents, anti-stretch mark agents, binding agents, agents regulating sebum production, lipolytic agents or agents stimulating lipolysis, anti-cellulite agents, antiperspirant agents, agents stimulating healing, coadjuvant healing agents, agents stimulating reepithelialization, coadjuvant reepithelialization agents, cytokine growth factors, calming agents, anti-inflammatory agents, anesthetic agents, agents acting on capillary circulation and/or microcirculation, agents stimulating angiogenesis, agents that inhibit vascular permeability, venotonic agents, agents acting on cell metabolism, agents to improve dermal-epidermal junction, agents inducing hair growth, hair growth inhibiting or retardant agents, muscle relaxants; antipollution and/or anti-free radical agents; lipolytic agents, venotonic agents, slimming agents, anticellulite agents, agents acting on the microcirculation; agents acting on the energy metabolism of the cells; cleaning agents, hair conditioning agents, hair styling agents, hair growth promoters, sunscreen and/or sunblock compounds, make-up agents, detergents, pharmaceutical drugs, emulsifiers, emollients, antiseptic agents, deodorant actives, dermatologically acceptable carriers, surfactants, abrasives, absorbents, aesthetic components such as fragrances, colorings/colorants, essential oils, skin sensates, cosmetic astringents, anti-acne agents, anti-caking agents, anti foaming agents, antioxidants, binders, biological additives, enzymes, enzymatic inhibitors, enzyme-inducing agents, coenzymes, chelating agents, plant extracts, plant derivatives, plant tissue extracts, plant seed extracts, plant oils, botanicals, botanical extracts, essential oils, marine extracts, agents obtained from a biofermentation process, mineral salts, cell extracts and sunscreens (organic or mineral photoprotective agents active against ultraviolet A and/or B rays), ceramides, peptides, buffering agents, bulking agents, chelating agents, chemical additives, colorants, cosmetic biocides, denaturants, drug astringents, external analgesics, film formers or materials, e.g., polymers, for aiding the film-forming properties and substantivity of the composition, quaternary derivatives, agents increasing the substantivity, opacifying agents, pH adjusters, propellants, reducing agents, sequestrants, skin bleaching and lightening agents, skin tanning agents, skin-conditioning agents (e.g., humectants, including miscellaneous and occlusive), skin soothing and/or healing agents and derivatives, skin treating agents, thickeners, and vitamins and derivatives thereof, peeling agents, moisturizing agents, curative agents, lignans, preservatives, UV absorbers, a cytotoxic, an antineoplastic agent, a fat-soluble active, suspending agents, viscosity modifiers, dyes, nonvolatile solvents, diluents, pearlescent aids, foam boosters, a vaccine, and their mixture.

The additional ingredient can be selected from the group consisting of sugar amines, glucosamine, D-glucosamine, N-acetyl glucosamine, N-acetyl-D-glucosamine, mannosamine, N-acetyl mannosamine, galactosamine, N-acetyl galactosamine, vitamin B3 and its derivatives, niacinamide, sodium dehydroacetate, dehydroacetic acid and its salts, phytosterols, salicylic acid compounds, hexamidines, dialkanoyl hydroxyproline compounds, soy extracts and derivatives, equol, isoflavones, flavonoids, phytantriol, farnesol, geraniol, peptides and their derivatives, di-, tri-, tetra-, penta-, and hexapeptides and their derivatives, KTTKS (SEQ ID NO:4), Pal-KTTKS (SEQ ID NO:2), carnosine, N-acyl amino acid compounds, retinoids, retinyl propionate, retinol, retinyl palmitate, retinyl acetate, retinal, retinoic acid, water-soluble vitamins, ascorbates, vitamin C, ascorbic acid, ascorbyl glucoside, ascorbyl palmitate, magnesium ascorbyl phosphate, sodium ascorbyl phosphate, vitamins their salts and derivatives, provitamins and their salts and derivatives, ethyl panthenol, vitamin B, vitamin B derivatives, vitamin B1, vitamin B2, vitamin B6, vitamin B12, vitamin K, vitamin K derivatives, pantothenic acid and its derivatives, pantothenyl ethyl ether, panthenol and its derivatives, dexpanthenol, biotin, amino acids and their salts and derivatives, water soluble amino acids, asparagine, alanine, indole, glutamic acid, water insoluble vitamins, vitamin A, vitamin E, vitamin F, vitamin D, mono-,di-, and tri-terpenoids, beta-ionol, cedrol, and their derivatives, water insoluble amino acids, tyrosine, tryptamine, butylated hydroxytoluene, butylated hydroxyanisole, allantoin, tocopherol nicotinate, tocopherol, tocopherol esters, pal-GHK, phytosterol, hydroxy acids, glycolic acid, lactic acid, lactobionic acid, keto acids, pyruvic acid, phytic acid, lysophosphatidic acid, stilbenes, cinnamates, resveratrol, kinetin, zeatin, dimethylaminoethanol, natural peptides, soy peptides, salts of sugar acids, Mn gluconate, Zn gluconate, particulate materials, pigment materials, natural colors, piroctone olamine, 3,4,4'- trichlorocarbanilide, triclocarban, zinc pyrithione, hydroquinone, kojic acid, ascorbic acid, magnesium ascorbyl phosphate, ascorbyl glucoside, pyridoxine, aloe vera, terpene alcohols, allantoin, bisabolol, dipotassium glycyrrhizinate, glycerol acid, sorbitol acid, pentaerythritol acid, pyrrolidone acid and its salts, dihydroxyacetone, erythrulose, glyceraldehyde, tartaraldehyde, clove oil, menthol, camphor, eucalyptus oil, eugenol, menthyl lactate, witch hazel distillate, eicosene and vinyl pyrrolidone copolymers, iodopropyl butylcarbamate, a polysaccharide, an essential fatty acid, salicylate, glycyrrhetinic acid, carotenoides, ceramides and pseudo-ceramides, a lipid complex, oils in general of natural origin such shea butter, apricot oil, onagre oil, prunus oil, palm oil, monoi oil, HEPES, procysteine, O-octanoyl-6-D-maltose, the disodium salt of methylglycinediacetic acid, steroids such as diosgenin and derivatives of DHEA, DHEA or dehydroepiandrosterone and/or a precursor or chemical or biological derivative, N-ethyloxycarbonyl-4-para-aminophenol, bilberry extracts; phytohormones; extracts of the yeast Saccharomyces cerevisiae, extracts of algae, extracts of soyabean, lupin, maize and/or pea, alverine and its salts, in particular alverine citrate, extract of butcher's broom and of horse chestnut, and mixtures thereof, a metalloproteinase inhibitor.

Further skin care and hair care active ingredients that are particularly useful can be found in SEDERMA commercial literature and on the website www.sederma.fr.

In any embodiment of the present invention, however, the additional ingredients useful herein can be categorized by the benefit they provide or by their postulated mode of action. However, it is to be understood that the additional ingredients useful herein can in some instances provide more than one benefit or operate via more than one mode of action. Therefore, classifications herein are made for the sake of convenience and are not intended to limit the additional ingredients to that particular application or applications listed.

The following known actives can be mentioned, as examples: betain, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Essenskin™ (Sederma), Moist 24™ (Sederma), Argireline™ (trade name of the acetyl hexapeptide-3 of Lipotec), spilanthol or an extract of *Acmella oleracea* known under the name Gatuline Expression™ (EP 1722864), an extract of *Boswellin serrata* known under the name Boswellin™, Deepaline PVB™ (Seppic), Syn-AKE™ (Pentapharm), Ameliox™, Bioxilift™ (Silab) or mixtures thereof.

Among other plant extracts which can be combined with the compound of the invention, there may more particularly be mentioned extracts of Ivy, in particular English Ivy *(Hedera Helix),* of Chinese thorowax (*Bupleurum chinensis),* of *Bupleurum Falcatum,* of arnica *(Arnica Montana L),* of rosemary (*Rosmarinus officinalis N),* of marigold (*Calendula officinalis),* of sage *(Salvia officinalis L),* of ginseng *(Panax ginseng),* of ginko biloba, of St.-John's-Wort *(Hyperycum Perforatum*), of butcher's-broom *(Ruscus aculeatus L),* of European meadowsweet (*Filipendula ulmaria L),* of big- flowered Jarva tea *(Orthosiphon Stamincus Benth),* of algae *(Fucus Vesiculosus),* of birch (*Betula alba),* of green tea, of cola nuts *(Cola Nipida),* of horse-chestnut, of bamboo, of spadeleaf (*Centella asiatica),* of heather, of fucus, of willow, of mouse-ear, of escine, of cangzhu, of *chrysanthellum indicum,* of the plants of the *Armeniacea* genus, *Atractylodis Platicodon, Sinnomenum*, *Pharbitidis, Flemingia*, of *Coleus* such as *C. Forskohlii, C. blumei, C. esquirolii, C. scutellaroides, C. xanthantus* and *C, Barbatus,* such as the extract of root of *Coleus barbatus,* extracts of *Ballote,* of *Guioa,* of *Davallia*, of *Terminalia,* of *Barringtonia,* of *Trema,* of *antirobia, cecropia, argania, dioscoreae* such as *Dioscorea opposita* or Mexican, extracts of *Ammi visnaga*, of *Centella asiatica* and *Siegesbeckia,* in particular *Siegesbeckia orientalis,* vegetable extracts of the family of *Ericaceae,* in particular bilberry extracts (*Vaccinium angustifollium*) or *Arctostaphylos uva ursi, aloe vera,* plant sterols (e.g., phytosterol), Manjistha (extracted from plants in the genus *Rubia,* particularly *Rubia Cordifolia),* and Guggal (extracted from plants in the genus *Commiphora*, particularly *Commiphora Mukul),* kola extract, chamomile, red clover extract, *Piper methysticum* extract *(Kava Kava* from SEDERMA (FR 2 771 002 and WO 99 / 25369), *Bacopa monieri* extract (Bacocalmine™ from SEDERMA, WO 99/40897) and sea whip extract, extracts of *Glycyrrhiza glabra,* of mulberry, of *melaleuca* (tea tree), of *Larrea divaricata*, of *Rabdosia rubescens,* of *euglena gracilis,* of *Fibraurea recisa Hirudinea,* of *Chaparral Sorghum*, of sun flower extract, of *Enantia chlorantha,* of Mitracarpe of *Spermacocea* genus, of *Buchu barosma*, of *Lawsonia inermis L.,* of *Adiantium Capillus-Veneris L.,* of *Chelidonium majus,* of *Luffa cylindrical*, of Japanese Mandarin *(Citrus reticulata Blanco* var. *unshiu),* of *Camelia sinensis,* of *Imperata cylindrical*, of *Glaucium Flavum*, of *Cupressus Sempenvirens,* of *Polygonatum multiflorum,* of *loveyly hemsleya*, of *Sambucus Nigra,* of *Phaseolus lunatus*, of *Centaurium*, of *Macrocystis Pyrifera,* of *Turner Diffusa,* of *Anemarrhena asphodeloides,* of *Portulaca pilosa,* of *Humulus lupulus,* of Coffea Arabica and *of Ilex Paraguariensis.*

Extraction from the plant may be performed using conventional engineering such as phenolic extraction, from any part of the plant such as the flower, seed, fruit, root, tubercle, leaf, pericarp and preferably rhizome. The extraction solvents may be selected from amongst water, propylene glycol, butylene glycol, glycerin, PEG-6 caprylic/capric glycerides, polyethylne glycol, methyl and/or ethyl esters, diglycols, cyclical polyols, ethoxylated or propoxylated diglycols, alcohols (methanol, ethanol, propanol, and butanol) or any mixture of these solvents. Plant extracts according to the present invention may also be obtained by other processes such as maceration, simple decoction, lixiviation, reflux extraction, supercritical extraction with CO₂, ultrasound or microwave extraction or counter-current techniques, or by plant cell culture engineering and/or fermentation. This list is not restrictive.

Suitable peptides can include, but are not limited to, di-, tri-, tetra-, penta-, and hexa- peptides and derivatives thereof. In one embodiment, the composition comprises from about 1x10-7% to about 20%, more preferably from about 1x10-6% to about 10%, even more preferably from about 1x10-5% to about 5%, by weight of additional peptide.

As used herein, "peptide" refers to peptides containing ten or fewer amino acids and their derivatives, isomers, and complexes with other species such as metal ions (e.g., copper, zinc, manganese, magnesium, and the like). As used herein, peptide refers to both naturally occurring and synthesized peptides. Also useful herein are naturally occurring and commercially available compositions that contain peptides.

Suitable dipeptides for use herein include but are not limited to Carnosine (beta-AH), YR, VW, NF, DF, KT, KC, CK, KP, KK or TT. Suitable tripeptides for use herein include, but are not limited to RKR, HGG, GHK, GKH, GGH, GHG, KFK, GKH, KPK, KMOK, KMO2K or KAvaK. Suitable tetrapeptides for use herein include but are not limited to RSRK (SEQ ID NO: 5), GQPR (SEQ ID NO: 6) or KTFK (SEQ ID NO: 7). Suitable pentapeptides include, but are not limited to KTTKS (SEQ ID NO: 4). Suitable hexapeptides include but are not limited to GKTTKS (SEQ ID NO: 8), VGVAPG (SEQ ID NO: 9) and of the type disclosed in FR 2854897 and US 2004/0120918.

Other suitable peptides for use herein include, but are not limited to lipophilic derivatives of peptides, preferably palmitoyl derivatives, and metal complexes of the aforementioned (e.g., copper complex of the tripeptide His-Gly-Gly). Preferred dipeptide derivatives include N-Palmitoyl-beta-Ala-His, N-Acetyl-Tyr-Arg-hexadecylester (CALMOSENSINE™ from SEDERMA, France, WO 9807744, US 6,372,717). Preferred tripeptide derivatives include N-Palmitoyl-Gly-Lys-His, (Pal-GKH from SEDERMA, France, WO 0040611), Pal-KMO2K, a copper derivative of His-Gly-Gly sold commercially as lamin, from Sigma, lipospondin (N-Elaidoyl-Lys-Phe-Lys) and its analogs of conservative substitution, N-Acetyl-Arg-Lys-Arg-NH2 (Peptide CK+), N-Biot-Gly-His-Lys (N-Biot-GHK from SEDERMA, WO0058347) and derivatives thereof. Suitable tetrapeptide derivatives for use herein include, but are not limited to N-palmitoyl-Gly-Gln-Pro-Arg (SEQ ID NO: 3) (from SEDERMA, France), suitable pentapeptide derivatives for use herein include, but are not limited to N-Palmitoyl-Lys-Thr-Thr-Lys-Ser (SEQ ID NO: 2) (available as MATRIXYL™ from SEDERMA, France, WO 0015188 and US 6,620, 419) N-Palmitoyl-Tyr-Gly-Gly-Phe-X with X Met (SEQ ID NO: 10) or Leu (SEQ ID NO: 11) or mixtures thereof. Suitable hexapeptide derivatives for use herein include, but are not limited to N-Palmitoyl-Val-Gly-Val-Ala-Pro-Gly (SEQ ID NO: 1) and derivatives thereof.

The preferred compositions commercially available containing a tripeptide or a derivative include Biopeptide-CL™ by SEDERMA (WO0143701), Maxilip™ by SEDERMA (WO 0143701), Biobustyl™ by SEDERMA. The compositions commercially available preferred sources of tetrapeptides include RIGIN™ (WO0043417), EYELISS™ (WO03068141), MATRIXYL™ RELOADED, and MATRIXYL 3000™ which contain between 50 and 500 ppm of palmitoyl-Gly-Gln-Pro-Arg (SEQ ID NO: 3), and carrier, proposed by SEDERMA, France (US2004/0132667).

The following marketed peptides can be mentioned as well as additional active ingredients: Vialox™, Syn-ake™ or Syn-Coll™ (Pentapharm), Hydroxyprolisilane CN™ (Exsymol), Argireline™, Leuphasyl™, Aldenine™, Trylgen™, Eyeseryl™, Serilesine™ or Decorinyl™ (Lipotec), Collaxyl™ or Quintescine™ (Vincience), BONT-L-Peptide™ (Infinitec Activos), Cytokinol™LS (Laboratoires Serobiologiques/Cognis), Kollaren™, IP2000™ or Meliprene™ (Institut Europeen de Biologie Cellulaire), Neutrazen™ (Innovations), ECM-Protect™ (Atrium Innovations), Timp-Peptide™ or ECM Moduline™ (lnfinitec Activos),

### Composition preparation

The compositions of the present invention are generally prepared by conventional methods such as are known in the art of making topical and oral compositions and compositions for injection. Such methods can typically be conducted in one or more steps, with or without heating, cooling, and the like.

The physical form of the compositions according to the invention is not important: they may be in any galenic form such creams, lotions, milk or cream ointments, gels, emulsions, dispersions, solutions, suspensions, cleansers, foundations, anhydrous preparations (sticks, in particular lipbalm, body and bath oils), shower and bath gels, shampoos and scalp treatment lotions, cream or lotion for care of skin or hair, make-up removing lotions or creams, sun-screen lotions, milks or creams, artificial suntan lotions, creams or milks, pre-shave, shave or aftershave creams, foams, gels or lotions, make-up, lipsticks, mascaras or nail varnishes, skin "essences," serums, adhesive or absorbent materials, transdermal patches, or powders, emollient lotion, milk or cream, sprays, oils for the body and the bath, foundation tint bases, pomade, emulsion, colloid, compact or solid suspension, pencil, sprayable or brossable formulation, blush, red, eyeliner, lipliner, lip gloss, facial or body powder, styling foams or gels, nail conditioner, lip balms, skin conditioners, moisturizers, hair sprays, soaps, body exfoliants, astringents, depilatories and permanent waving solutions, antidandruff formulations, anti-sweat and antiperspirant compositions, nose sprays and so on. These compositions can also be presented in the form of lipsticks intended to apply color or to protect the lips from cracking, or of make-up products for the eyes or tints and tint bases for the face. Compositions in accordance with the invention include cosmetics, personal care products and pharmaceutical preparations. The present invention may also be applied on animal skin and/or appendages. One can also consider a composition in the shape of foam or in the form of compositions for aerosol also including a propellant agent under pressure.

Cosmetic compositions according to the invention may also be for orodental use, for example, toothpaste. In that case, the compositions may contain the usual adjuvants and additives for compositions for oral use and, in particular, surfactants, thickening agents, moisturizing agents, polishing agents such as silica, various active substances such as fluorides, particularly sodium fluoride, and, possibly, sweetening agents such as saccharin sodium.

The compound according to the present invention may be in the form of solution, dispersion, emulsion, paste, or powder, individually or as a premix or in vehicles individually or as a premix in vectors such as macro-, micro-, or nanocapsules, macro-, micro- or, nanospheres, liposomes, oleosomes or chylomicrons, macro-, micro-, or nanoparticles or macro-, micro or nanosponges, spores or exines, micro or nano emulsions or adsorbed on organic polymer powders, talcs, bentonites, or other inorganic or organic supports.

The compound according to the present invention may be used in any form whatsoever, in a form bound to or incorporated in or absorbed in or adsorbed on macro-, micro-, and nanoparticles, or macro-, micro-, and nanocapsules, for the treatment of textiles, natural or synthetic fibres, wools, and any materials that may be used for clothing or underwear for day or night intended to come into contact with the skin, handkerchiefs or cloths, to exert their cosmetic effect via this skin/textile contact and to permit continuous topical delivery.

### Method of topical cosmetic or dermopharmaceutical treatment

The present invention also concerns a topical treatment method to improve the general condition of the skin involving topical application to the skin of an effective amount of the composition of the invention as recited above. More specifically:
- to prevent and/or treat the signs of intrinsic and extrinsic skin ageing;
- to prevent and/or treat skin dehydration;
- to prevent and/or treat skin sagging and/or improve tone and/or firmness and/or elasticity and/suppleness of the skin;
- to prevent and/or treat skin atrophy and/or improve the density of the dermis and epidermis;
- to give or return volume to the dermis and epidermis;
- For stimulating the expansion of adipose tissue.
- to lighten the skin;
- to prevent and/or treat skin roughness;
- to prevent and/or treat degradation of the skin due to the effects of oxidation;
- to prevent and/or treat hair loss;
- to prevent and/or treat glycation of molecules in the skin;
- to prevent and/or treat acne;
- To prevent and/or treat inflammatory states.

The composition according to the invention may be applied locally onto areas of the face, lips, neck, neckline, hands, feet, head or body. One of the major advantages of the present invention resides in the ability whenever necessary or desirable to be able to apply local selective "gentle" treatments through this topical, non-invasive method of application. In the case of anti-wrinkle use for example it may be applied very locally using a syringe or micro-canula.

It is also possible, however, to consider a composition containing the compound according to the invention intended to be injected subcutaneously.

According to other specific features the treatment method according to the invention can be combined with one or more other treatment methods targeting the skin such as luminotherapy, aromatherapy or heat treatments.

According to the invention, devices with several compartments or kits may be proposed to apply the method described above which may include for example and non-restrictively, a first compartment containing a composition including the invention compound, and in a second compartment a composition containing another active ingredient and/or excipient, the compositions contained in the said first and second compartments in this case being considered to be a combination composition for simultaneous, separate or stepwise use in time, particularly in one of the treatment methods recited above.

### Examples

### 1/ Formulation of an active ingredient according to the invention

The compounds of the invention can be dissolved in a hydrophobic matrix (e.g. based on triglycerides) at a concentration typically of 2% w/w (=with regard to the total weight of the composition), corresponding to 20 000 ppm, in order to form an active ingredient that can be used in the manufacture of cosmetic products (as disclosed in below galenic examples of point 3 /).

### 2/ In vitro TESTS

The compounds of the invention demonstrate various activity cosmetics, affecting several domains of the skin physiology.

### 2/1 Activity of the invention compound on adipocyte differentiation:

Some cosmetic compounds seek to encourage the installation of the subcutaneous fat for better aesthetics and greater volume. In this regard, *in vitro* tests were implemented in order to observe the increase of adipocyte differentiation on cultured pre-adipocytes (with glycerol-3-phosphate dehydrogenase (G3PDH) as the key enzyme of this differentiation).

### Effect of the compounds according to the invention on the G3PDH activity

Principle: 3T3-L1 cells were grown to sub-confluence, then induced to differentiate with the appropriate cocktail in the presence or absence of geranyl geranyl hexenoate acetate (GGHE) or geranyl geranyl octadieneoate acetate (GGOE) at different concentrations. After three days of incubation, the cocktail of differentiation is replaced by a new culture medium in the presence or absence of GGHE or GGOE. After 3 days of incubation, the cell layers were recovered and G3PDH activity is assayed, and then divided by the number of cells.

**Table 1: Effect of GGHE and GGOE on G3PDH activity by cell in the adipocyte culture**

| **Product** | **Concentration** | **% Change/control (N=2)** |
|---|---|---|
| **GGHE** | 5µM | +77%; p<0.01 |
| | 10µM | +97%; p<0.01 |
| **GGOE** | 5µM | +83%; p<0.01 |
| | 10µM | +84%; p<0.01 |

| | | |
|---|---|---|
| Pioglytazone (positive control) 10µM: +224%; p<0.01 | | |

Thus, the compound of the invention, here the GGOE or GGHE at 5 and 10µM, significantly stimulates adipocyte differentiation. According to this result, the compounds can be recommended in any cosmetic composition for promoting expansion and/or formation of subcutaneous adipose tissue and/or stimulate lipogenesis and/or adipogenesis and/or adipocyte differentiation.

### 2/2 Activity of the compound according to the invention on melanogenesis of human melanocytes in monolayers:

The cosmetics industry is looking for compounds with depigmenting (whitening, skin depigmenting and lightening, elimination or mitigation of freckles, age spots etc.). *In vitro,* it is possible to demonstrate such an effect by measuring the melanin synthesized by melanocytes cultures that had or not been in contact with the compounds to be evaluated.

### Effect of the compounds according to the invention on the quantity of synthetized melanin

Principle: Human melanocytes are cultured and contacted or not with the geranylgeranyl ethyl hexenoate (GGHE) or the geranylgeranyl methyl pentenoate (GGPM), at different concentrations, for 5 days. After incubation, the melanin content is measured in the cell homogenates, then divided by the cell number.

**Table 2: Effect of GGHE and GGPM on melanin synthesis with regard to cell in a melanocyte cuture**

| **Product** | **Concentration** | **Melanin (% Change/Control)** | |
|---|---|---|---|
| **GGHE** | 1ppm | -10% | p < 0.01 |
| | 3ppm | -22% | p < 0.01 |
| | 5ppm | -30% | p < 0.01 |
| | 7ppm | -37% | p < 0.01 |
| | 10ppm | -47% | p < 0.01 |
| **GGPM** | 1ppm | -6% | p < 0.01 |
| | 3ppm | -24% | p < 0.01 |
| | 5ppm | -36% | p < 0.01 |
| | 7ppm | -44% | p < 0.01 |
| | 10ppm | -48% | p < 0.01 |

| | | | |
|---|---|---|---|
| Arbutin (positive control) 0.06% = -57%; p<0.01 | | | |

A significant and dose dependant decrease of the melanin synthesis by the human melanocyte is observed in the presence of the two GGHE and GGPM compounds of the invention.

### 2/3 Anti-radical and anti-oxydant effects of the compounds of the invention:

Oxygen free radicals are produced during normal physiological processes such as breathing or the inflammatory response, but may cause serious cell damage involved in skin aging if produced in excessive amounts (oxidative stress), then passing over the antioxidant defense mechanisms. Therefore, in particular according to this, anti-radical compounds, and thus the invention compounds, can present an interest in the anti-aging cosmetic compositions.

Principle: The ORAC test (Oxygen-Radical Absorbance Capacity) measures the oxidative degradation of a fluorescent molecule (fluorescein). Degradation of fluorescein by peroxyl radicals generated by AAPH (2,2 '-azobis (2-amidinopropane) dichloride) induces a decrease in fluorescence that can be measured by a spectrofluorometer, during a degradation kinetic.

An antioxidant protects fluorescein against this radical degradation. The fluorescence decrease will be smaller and slower in the presence of the antioxidant than in its absence. The comparison between the two kinetic parameters enables to calculate a protection percentage against free radical degradation.

**Table 3: Effect of GGHE, GGOE and GGPM on fluorescein degradation by peroxyl radicals**

| | **Control** | **GGA 10ppm** | **GGPE 10ppm** | **GGHE 10ppm** | **GGOE 10ppm** |
|---|---|---|---|---|---|
| **% of protection** | REF | -3% | 43% | 32% | 42% |
| **Student *t* Test** | | nsd | p<0.01 | p<0.01 | p<0.01 |

| | | | | | |
|---|---|---|---|---|---|
| Positive control = Trolox 2µM = 47% (p<0.01); nsd = non significative data | | | | | |

A significant protection against peroxyl radicals in the presence of GGPE, GGHE and GGOE, each at 10ppm, is observed. The compounds according to the invention have in this test an antiradical activity higher to geranylgeranylacetone (GGA), known anti-aging molecule (the subject matter of a patent of the Applicant).

### 2/4 Keratinocyte protection against UVB by the invention compounds:

Skin is very sensitive to UV radiations. Overexposure to the sun (which includes UV A and B in its spectrum) can lead to premature aging of the skin, and even to cancer process. Thus, it is important for the cosmetics industry to find compounds able to counteract the negative effects of UV radiations. Keratinocytes in monolayer after UVB irradiation (12 to 240mJ/cm ²) develop on their surface many buds, separate themselves from the culture support and are damaged that can go up to death. A cell count at various times after UVB irradiation can be used to evaluate the cell viability of the culture. This model is widely used in cosmetics to evaluate the protective effect of various compounds against the deleterious effects of UVB stress.

Principle: Keratinocytes are cultured in 35mm Petri dish. Just at confluence, the cells are contacted or not with the GGHE and GGOE invention compounds for 24 hours. UVB irradiation (50mJ/cm ²) is performed the next day, and then the cells are or not contacted with the invention compounds. 6 days later, the cells are counted to determine cell viability.

**Table 4: Quantification of the number of cells post UVB stress**

| | | **Number of cells (x10⁶)/ 35mm Petri dish** | | | |
|---|---|---|---|---|---|
| | **Concentration** | **mean (n=3)** | **Ecart type** | **% change /control** | **Stat (t student)** |
| **Control** | - | 0,43 | 0,014 | Ref | Ref |
| **GGHE** | **1ppm** | 0,671 | 0,079 | +56% | p<0,01 |
| | **2,5ppm** | 0,674 | 0,075 | +57% | p<0,01 |
| **Control** | - | 0,447 | 0,05 | Ref | Ref |
| **GGOE** | **1ppm** | 0,639 | 0,162 | +43% | p<0.05 |
| | **2,5ppm** | 0,658 | 0,039 | +47% | p<0,01 |

The GGHE and GGOE compounds according to the invention, at concentations of 1 and 2,5ppm have a significant protective effect against the UVB stress effects.

### 2/5 Action on decorin, molecule of the dermis:

Decorin is a leucine-rich proteoglycan involved in the assembly of the dermis, by binding to collagen fibers and tropoelastin. The quality of this assemblage reflects the skin mechanical properties: elasticity, compressibility and firmness.

Principle: Normal human fibroblasts were brought to confluence and then placed or not in contact with the GGPE, GGOE and GGHE invention compounds, at 5ppm, for 3 days. After this incubation, decorin content was assayed by ELISA in cell homogenates, and divided by the number of cells.

**Table 5: Effect of GGHE, GGOE and GGPE on the biosynthesis of decorin with regard to cell in a fibroblast culture**

| | **Control** | **GGPE 5ppm** | **GGHE 5ppm** | **GGOE 5ppm** |
|---|---|---|---|---|
| **Stimulation %** | REF | +62% | +56% | +54% |
| **Student t test** | | p<0.01 | p<0.01 | p<0.01 |

A significant increase in the decorin synthesis by human fibroblast is observed in the presence of 5ppm of the GGHE, GGPE and GGOE compounds of the invention.

### 2/6 Action on nidogen 1, molecule of the dermal-epidermal junction:

Nidogen 1 is a key component of the basal membrane. It stabilizes and strengthens the basal membrane by linking laminin to collagen 4.

Principle: Normal human fibroblasts were brought to confluence and then placed or not in contact with the GGPE, GGOE and GGHE compounds of the invention at 5ppm for 3 days. After this incubation, the content Nidogen 1 is determined by ELISA in cell homogenates, and divided by the number of cells.

**Table 6: Effect of GGHE, GGOE and GGPE on nidogen 1 biosynthesis by cell in a fribroblast culture**

| | **Control** | **GGPM 5ppm** | **GGPE 5ppm** | **GGHE 5ppm** | **GGOE 5ppm** |
|---|---|---|---|---|---|
| **Stimulation %** | REF | +43% | +117% | +86% | +60% |
| **Student *t* test** | | p<0.01 | p<0.01 | p<0.01 | p<0.01 |

A significant increase of the nidogen 1 synthesis by human fibroblast is observed in the presence of 5ppm of each of the four compounds according to the invention (GGPM, GGPE, GGHE and GGOE). Other activities can of course be highlighted according to other appropriated tests, in particular using *in vivo* tests.

### 2/7 Influence of the GGOE on the syndecan 1 synthesis by human keratinocytes

Syndecan 1 is a small proteoglycan transmembran protein that is strongly implicated in keratinocyte activation and cohesion. The production of syndecan 1 diminishes with age resulting in a lack of cohesion at the level of epidermis (in the supra basal layers).

**Principle:** Human keratinocytes are cultivated in a growth medium. After 6 days of incubation, the cells are contacted or not with the tested products (GGOE and GGA) at 3, 5 or 7 ppm for 48h. After incubation, the supernatants are taken and the syndecan 1 content is assayed by ELISA in these supernatants. The cell layers are sonicated and the cell number is evaluated by Hoechst method.

**Table 7: Influence of GGOE (and compared to GGA) on syndecan 1 synthesis in human keratinocytes supernatants (n=3)**

| | **Concentration** | **Syndecan 1 (pg / 10⁶ cell)** | **Change (%)** |
|---|---|---|---|
| **Control** | - | 136.0 ± 10.0 | Reference |
| **GGA** | 3ppm | 141.3 ± 12.9 | +4%; *nsd* |
| | 5ppm | 155.7 ± 14.4 | +14%; *nsd* |
| | 7ppm | 183.4 +/- 21.9 | +35%; *p<0.05* |
| **Control** | - | 136.4 ± 19.0 | Reference |
| **GGOE** | 3ppm | 353.4 ± 57.2 | +159%; *p<0.01* |
| | 5ppm | 424.3 ± 69.1 | +211%; *p<0.01* |
| | 7ppm | 435.9 +/- 18.3 | +220%; *p<0.01* |

The results show that there is a significant and dose dependent stimulation of the synthesis of syndecan 1 in human keratinocyte in the presence of GGOE (stimulation > 200% in the presence of 7ppm of GGP).

Comparatively, the GGA presents a low stimulating activity of syndecan 1 syntesis (stimulation < 50% in the presence of 7 ppm of GGA).

Therefore, GGOE can boost the synthesis of syndecan 1 in keratinocytes to reinforce the skin cohesion.

### 3/ Galenic examples

### 3/1 Gel form for face

### Ingredient according to the invention:

Formulation comprising 2% w/w of the compound of the invention in a hydrophobic matrix

### Examples of other additionnal/optional ingredients:

RIGIN^{™} is an ingredient product marketed by SEDERMA (FR 2788777 and WO 00/433417) improving the elasticity and firmness of skin, reinforcing hydration and smoothing skin.
REVIDRAT^{™} is an ingredient product marketed by SEDERMA which improves the epidremal cohesion and its hydration.
LUMISKIN^{™} is an ingredient product marketed by SEDERMA (WO 2004/024695) for lightening skin complexion.
SUBLISKIN^{™} is an ingredient product marketed by SEDERMA (WO 2009/055663) for hydrating and smoothing skin while enabling the skin to better resist to externa agressions.
MATRIXYL3000™ is an anti-wrinkle ingredient product marketed by WEDERMA (WO 2005/048968) which helps to repare cutaneous damages caused by aging.

| **GEL FORM FOR FACE** | | **Weight %** | | | | | |
|---|---|---|---|---|---|---|---|
| **Ingredients** | **INCI Name** | **Gel n°1** | **Gel n°2** | **Gel n°3** | **Gel n°4** | **Gel n°5** | **Gel n°6** |
| **Phase A** | | | | | | | |
| H₂O | Water | Qsp 100 | Qsp 100 | Qsp 100 | Qsp 100 | Qsp 100 | Qsp 100 |
| Cetyl hydroxyethylcellulose | Cetyl hydroxyethylcellulose | 0.30 | 0.30 | 0.30 | 0.30 | 0.30 | 0.30 |

| **Phase B** | | | | | | | |
|---|---|---|---|---|---|---|---|
| Ultrez 10 | Carbomer | 0.40 | 0.20 | 0.20 | 0.20 | 0.20 | 0.20 |
| H₂O | Water | 20.00 | 20.00 | 20.00 | 20.00 | 20.00 | 20.00 |

| **Phase C** | | | | | | | |
|---|---|---|---|---|---|---|---|
| Glycerin | Glycerin | 3.00 | 3.00 | 3.00 | 3.00 | 3.00 | 3.00 |
| Panstat | Ethyl & Methyl & Propyl parabens | 0.30 | 0.30 | 0.30 | 0.30 | 0.30 | 0.30 |

| **Phase D** | | | | | | | |
|---|---|---|---|---|---|---|---|
| Formula comprising 2% w/w of the invention compound in an hydrophobic matrix | | 3.00 | 3.00 | 3.00 | 3.00 | 3.00 | 3.00 |
| REVIDRAT™ | | | | 3.00 | | | |
| LUMISKIN™ | Caprylic/Capric Triglyceride (and) | | | | 3.00 | | |
| | Diacetyl Boldine | | | | | | |
| Marcol 82 | Mineral oil | 2.00 | 4.00 | 2.00 | 1.00 | 2.00 | 1.00 |
| Crillet 1 | Polysorbate 20 | 1.00 | 1.00 | 1.00 | 1.00 | 1.00 | 1.00 |
| Crodamol AB | C12-15 Alkyl Benzoate | 2.00 | - | 2.00 | 2.00 | 2.00 | 2.00 |
| Pemulen TR2 | C 10-30 Alkyl Acrylate cross polymer | 0.30 | 0.30 | 0.30 | 0.30 | 0.30 | 0.30 |

| **Phase E** | | | | | | | |
|---|---|---|---|---|---|---|---|
| Sorbate de potassium | Potassium Sorbate | 0.10 | 0.10 | 0.10 | 0.10 | 0.10 | 0.10 |

| **Phase F** | | | | | | | |
|---|---|---|---|---|---|---|---|
| H₂O | Water | 5.00 | 5.00 | 5.00 | 5.00 | 5.00 | 5.00 |
| NaOH 10N | Sodium Hydroxyde | 0.50 | 0.50 | 0.50 | 0.50 | 0.50 | 0.50 |

| **Phase G** | | | | | | | |
|---|---|---|---|---|---|---|---|
| RIGIN™ | Water (Aqua) - Glycerin - Steareth 20 - Palmitoyl Tetrapeptide-7 | | 3.00 | | | | |
| MATRIXYL3000™ | Glycerin (and) Water (Aqua) (and) Butylene Glycol (and) Carbomer (and) Polysorbate-20 (and) Palmitoyl Oligopeptide (and) Palmitoyl Tetrapeptide-7 | | | | | 3.00 | |
| SUBLISKIN™ | Sinorhizobium Meliloti Ferment (and) Cetyl Hydroxyethylcellulose (and) Lecithin | | | | | | 3.00 |

| | | | | | | | |
|---|---|---|---|---|---|---|---|
| **Protocol:** Disperse Phase A under stirring. Sprinkle Ultrez 10 in water, let swell for 30 minutes. Heat the Phase C until complete dissolution. Mix Phase A with Phase B. Add Phase C in Phase (B+A). Add Phase D under stirring in Phase (A+B+C). Add Phase E. Neutralize with Phase F. Add Phase G if appropriate and mix. **Gel n°1 features:** Volumizing effect by «lipofilling like», anti-aging (antioxydant), lightening and firming. **Gel n°2 features:** The additional active ingredient RIGIN^{™} reinforces the anti-aging properties of the active ingredient according to the invention, in particular improving the mechanical properties of the skin and its hydration. **Gel n°3 features:** The additionnal active ingredient REVIDRAT^{™} reinforces the epidermal cohesion and increases the effects of the invention ingredient (volumizing effect by stimulation of the adiposgenesis and lightening effect). **Gel n°4 features:** The additionnal active ingredient LUMISKIN^{™} reinforces the lightening action of the invention compound. **Gel n°5 features:** The invention compound associated with the MATRIXIL TM 3000 gives to this gel both both protective and repairing properties against aging. **Gel n°6 features:** to the properties of the invention compound, SUBLISKIN^{™} adds confort and hydration effects to the skin. | | | | | | | |

### 3/2 Cream form for face

### Ingredient according to the invention:

Formulation comprising 2% w/w of the compound of the invention in a hydrophobic matrix

### Examples of other additionnal/optional ingredients:

KOMBUCHA^{™} is an ingredient product marketed by SEDERMA (FR 0209710) in particular used for densifying the adipocyte population and also to promote skin complexion.
STEROCARE^{™} is an anti-aging active ingredient marketed by SEDERMA (WO 99/18927) especially recommended for mature skin.
IDEALIFT^{™} is an anti-aging and calming active ingredient, containing the Tyr-Arg lipopeptide, marketed by SEDERMA (FR 09/53444). It improves the mechanical properties of the skin, especially to combat skin flaccidity (that is to say the firmness), for example at the jowls of the face. It is also an active known for its muscle relaxant properties and anti-rednesses. It stimulates the synthesis of elastic fibers.

Niacinamide, Retinol, Resveratrol: anti-aging actives, in particular anti-wrinkle actives.

| **CREAM FORM FOR FACE** | | **Weight %** | | | | | | |
|---|---|---|---|---|---|---|---|---|
| **Ingredients** | **INCI Names** | **n°1** | **n°2** | **n°3** | **n°4** | **n°5** | **n°6** | **n°7** |
| **Phase A** | | | | | | | | |
| H₂O | Water | qsp100 | qsp100 | qsp100 | qsp100 | qsp100 | qsp100 | qsp100 |
| Ultrez 10 | Carbomer | 0.40 | 0.40 | 0.40 | 0.40 | 0.40 | 0.40 | 0.40 |

| **Phase B** | | | | | | | | |
|---|---|---|---|---|---|---|---|---|
| Glycerin | Glycerin | 3.00 | 3.00 | 3.00 | 3.00 | 3.00 | 3.00 | 3.00 |
| Panstat | Ethyl & Methyl & Propyl parabens | 0.30 | 0.30 | 0.30 | 0.30 | 0.30 | 0.30 | 0.30 |

| **Phase C** | | | | | | | | |
|---|---|---|---|---|---|---|---|---|
| Polawax GP 200 | Cetearyl Alcohol & polysorbate 20 | 1.00 | 1.00 | 1.00 | 1.00 | 1.00 | 1.00 | 1.00 |
| Crodacol CS 90 | Cetearyl Alcohol | 1.00 | 1.00 | 1.00 | 1.00 | 1.00 | 1.00 | 1.00 |
| Crodamol STS | PPG-3 Benzyl Ether Myristate | 1.00 | 1.00 | 1.00 | 1.00 | 1.00 | 1.00 | 1.00 |
| DC 200 5 cps | Dimethicone | 2.50 | 2.50 | 2.50 | 2.50 | 2.50 | 2.50 | 2.50 |
| Crodamol TN | Isotridecyl Isononanoate | 5.00 | 5.00 | 5.00 | 5.00 | 5.00 | 5.00 | 5.00 |

| **Phase D** | | | | | | | | |
|---|---|---|---|---|---|---|---|---|
| Formula comprising 2% w/w of the invention compound in an hydrophobic matrix | | 4.00 | 3.00 | 3.00 | 3.00 | 3.00 | 3.00 | 3.00 |
| Retinol | | | | | | | 0.1 | |
| Resveratrol | | | | | | | | 0.5 |

| **Phase E** | | | | | | | | |
|---|---|---|---|---|---|---|---|---|
| Potassium sorbate | Potassium sorbate | 0.10 | 0.10 | 0.10 | 0.10 | 0.10 | 0.10 | 0.10 |

| **Phase F** | | | | | | | | |
|---|---|---|---|---|---|---|---|---|
| NaOH 30% | Sodium hydroxyde | 0.40 | 0.40 | 0.40 | 0.40 | 0.40 | 0.40 | 0.40 |
| H₂O | Water | 4.00 | 4.00 | 4.00 | 4.00 | 4.00 | 4.00 | 4.00 |

| **Phase G** | | | | | | | | |
|---|---|---|---|---|---|---|---|---|
| KOMBUCHA™ | Saccharomyc es/Xylinum Black Tea Ferment, Glycerin, Hydroxyethyl cellulose | - | 3.00 | - | | | | |
| STEROCARE™ | Trifolium Pratense (Clover) Flower Extract, Glycerin, Butylene Glycol, Lecithin | - | | 3.00 | | | | |
| IDEALIFT™ | Butylene Glycol-Water-Sorbitan Laurate-Hydroxyethyl cellulose-Acetyl Dipeptide-1 Cetyl Ester | - | - | | 3.00 | | | |
| Niacinamide 10% in water | | | | | | 10.00 | | |

| **Phase H** | | | | | | | | |
|---|---|---|---|---|---|---|---|---|
| Fragrance | Fragrance | 0.10 | 0.10 | 0.10 | 0.10 | 0.10 | 0.10 | 0.10 |

| | | | | | | | | |
|---|---|---|---|---|---|---|---|---|
| **Protocol:** Weigh Phase A and let swell for 30 minutes. Then heat Phase A to 75C° in a water bath. Heat the Phase B until dissolution. Add Phase B into Phase A. Heat Phase C in a water bath at 75C °. While stirring, add Phase C to Phase (A+B). Extemporaneously add Phase D. Add Phase E, mix thoroughly. Neutralize with Phase F around 55 °C. Add Phase G, then Phase H, mix thoroughly. **Cream n°1 properties:** in particular a volumizing action by "lipofilling like", anti-aging (anti-oxidant), brightening/lightening and firming. **Cream n°2 properties:** the anti-aging and oleodensifying effects of the invention compound are enhanced by the association with the Kombucha^{™}. **Cream n°3 properties:** it reinforces the anti-aging properties for mature skin, thanks to Sterocare^{™}. **Cream n°4 properties:** the described effects of the invention are associated to the calming and anti-redness effecst of Idealift^{™}. **Cream n°5 properties:** the anti-aging treating effect of niacinamide is combined with the intrinsic properties of the compound of the invention. **Cream n°6 properties:** the anti-aging treating effect of retinol is combined with the intrinsic properties of the compound of the invention. **Cream n°7 properties:** resveratrol associated with compound of the invention will enable to obtain a skin lighter with fewer wrinkles and more flexible. | | | | | | | | |

### 3/3 Cream form for the body

### Ingredient according to the invention:

Formulation comprising 2% w/w of the compound of the invention in a hydrophobic matrix

### Examples of other additionnal/optional ingredients:

BIO-BUSTYL^{™} is an ingredient product marketed by SEDERMA (FR2668365) stimulating the cell metabolism and thus dynamising the skin, used in particular in cosmetic products for women bust. Tocopherol or vitamine E : anti-radical and antioxydant properties.
O.D.A. WHITE^{™} is an ingredient product marketed by SEDERMA (WO 94/07837) indicated for lightening the skin by melanin synthesis reduction.

| **CREAM FORM FOR BODY** | | **Weight %** | | | |
|---|---|---|---|---|---|
| **Ingredients** | **INCI names** | **n°1** | **n°2** | **n°3** | **n°4** |
| **Phase A** | | | | | |
| H₂O | Water | qsp 100 | qsp 100 | qsp 100 | qsp 100 |
| Ultrez 10 | Carbomer | 0.40 | 0.40 | 0.40 | 0.40 |

| **Phase B** | | | | | |
|---|---|---|---|---|---|
| Glycerine | Glycerin | 3.00 | 3.00 | 3.00 | 3.00 |
| Panstat | Ethyl & Methyl & Propyl parabens | 0.30 | 0.30 | 0.30 | 0.30 |

| **Phase C** | | | | | |
|---|---|---|---|---|---|
| Crill 3 | Sorbitan Stearate | 2.00 | 2.00 | 2.00 | 2.00 |
| Marcol 82 | Mineral oil | 4.00 | 2.00 | 6.00 | 4.00 |
| Cromollient DP3A | PPG 3 Myristyl Ether Adipate | 1.00 | 1.00 | 2.00 | 1.00 |
| Cithrol GMS AS | Glyceryl stearate & PEG 100 stearate | 3.00 | 3.00 | 3.00 | 3.00 |

| **Phase D** | | | | | |
|---|---|---|---|---|---|
| Formula comprising 2% w/w of the invention compound in an hydrophobic matrix | | 5.00 | 3.00 | 3.00 | 4.00 |
| O.D.A. white™ | Octadecenedioic Acid | | | 1.00 | |
| Tocopherol | | | | | 0.5 |

| **Phase E** | | | | | |
|---|---|---|---|---|---|
| Potassium sorbate | Potassium sorbate | 0.10 | 0.10 | 0.10 | 0.10 |

| **Phase F** | | | | | |
|---|---|---|---|---|---|
| NaOH 30% | Sodium hydroxide | 0.40 | 0.40 | 0.40 | 0.40 |
| H₂O | Water | 4.00 | 4.00 | 4.00 | 4.00 |

| **Phase G** | | | | | |
|---|---|---|---|---|---|
| Bio-bustyl™ | Rahnella Soy Protein Ferment, Palmitoyl Oligopeptide | - | 3.00 | | |

| **Phase H** | | | | | |
|---|---|---|---|---|---|
| Parfum | Fragrance | 0.10 | 0.10 | 0.10 | 0.10 |

| | | | | | |
|---|---|---|---|---|---|
| **Protocol:** Weigh Phase A and let it swallow for 30 minutes. Heat Phase A at 75C° using a water bath. Heat Phase B until dissolution. Add Phase B into Phase A. Heat Phase C at 75C °in a water bath. While stirring, add Phase C to Phase (A+B) and extamporaneously add Phase D. Add Phase E, mix thoroughly. Neutralize with Phase F around 55°C, homogenise thorouhgly. Add Phase G if appropriate, then Phase H, mix thoroughly. **Cream n°1 Properties:** adapted to the buttocks with a high active concentration for a high « lipofilling like» and firming volumizing effect. **Cream n°2 Properties:** adapted for the bust with in addition to the properties of the compound of the invention, the dynamising and firming effect of the Bio-bustyl^{™} ingredient. **Cream n°3 Properties:** oily formula for hands particularly adapted for treating yellow spots appearing with aging. **Cream n°4 Properties: the** antioxydant action of the invention compound is reinforced by the presence of tocopherol, this cream being thus adapted to protection against stress. | | | | | |

### 3/4 Serum form

### Ingredient according to the invention:

Formulation comprising 2% w/w of the compound of the invention in a hydrophobic matrix

### Examples of other additionnal/optional ingredients:

LUMISPHERE^{™} is an ingredient product marketed by SEDERMA (WO04/024695). It is Association of polymethylmethacrylate microencapsulated diacetylboldine (DAB) and manganese titanium dioxide (TiO2Mn). TiO2Mn provides the skin with an instant even, luminous and mat complexion with no white residue when DAB brings a physiological lightening effect.
REVIDRAT^{™} is an ingredient product marketed by SEDERMA, which improves the epidermal cohesion and its hydration.
EVERMAT^{™} is an ingredient product marketed by SEDERMA (WO 2007/029187), which decreases the sebum secretion and thus participates in the treatment of oily skin.
HALOXYL^{™} is an ingredient product marketed by SEDERMA (WO 2005/102266) which improves the eye contour by reducing rings under the eyes.

| **SERUM FORM** | | **Weight %** | | | | |
|---|---|---|---|---|---|---|
| **Ingredients** | **INCI name** | **Serum n°1** | **Serum n°2** | **Serum n°3** | **Serum n°4** | **Serum n°5** |
| **Phase A** | | | | | | |
| Optasens G 40 | Carbomer | 0.25 | 0.25 | 0.25 | 0.25 | 0.25 |
| H₂O | Water | Qsp100 | Qsp100 | Qsp100 | Qsp100 | Qsp100 |

| **Phase B** | | | | | | |
|---|---|---|---|---|---|---|
| Butylene Glycol | Butylene Glycol | 3.00 | 3.00 | 3.00 | 3.00 | 3.00 |
| Phenoxyethanol | Phenoxyethanol | 0.20 | 0.20 | 0.20 | 0.20 | 0.20 |

| **Phase C** | | | | | | |
|---|---|---|---|---|---|---|
| Crillet 1 | Polysorbate 20 | 0.50 | 0.50 | 0.50 | 0.50 | 0.50 |
| DC 245 | Cyclopentasiloxane | 1.00 | 1.00 | 1.00 | 1.00 | 1.00 |
| Crodamol CAP | Cetearyl Ethylhexanoate | 2.00 | 2.00 | - | 2.00 | 2.00 |
| | | | | | | |
| Crodamol STS | PPG-3 Benzyl Ether Myristate | 0.50 | 0.50 | 0.50 | 0.50 | 0.50 |
| Pemulen TR2 | Acrylates/C 10-30 Alkyl Acrylates cross polymer | 0.20 | 0.20 | 0.20 | 0.20 | 0.20 |
| Formula comprising 2% w/w of the invention compound in an hydrophobic matrix | / | 5.00 | 2.00 | 2.00 | 2.00 | 2.00 |
| REVIDRAT™ | | | | 2.00 | | |

| **Phase D** | | | | | | |
|---|---|---|---|---|---|---|
| Potassium sorbate | Potassium Sorbate | 0.10 | 0.10 | | | |

| **Phase F** | | | | | | |
|---|---|---|---|---|---|---|
| H₂O | Water | 4.00 | 4.00 | | | |
| NaOH 30 % | Sodium Hydroxyde | 0.45 | 0.45 | | | |

| **Phase E** | | | | | | |
|---|---|---|---|---|---|---|
| LUMISPHERE™ | Water (Aqua) (and) Titanium Dioxide (and) Polysorbate-20 (and) Cetyl Hydroxyethylcellulos e (and) polymethylmethacryl ate (and) Trilaurin (and) Diacetyl Boldine | | 4.00 | | | |
| EVERMAT™ | Butylene Glycol (and) Enantia Chlorantha Extract (and) Oleanolic Acid | | | | 4.00 | |
| HALOXYL™ | Water (Aqua) (and) Glycerin (and) Steareth-20 (and) N-Hydroxysuccinimide (and) Chrysin (and) Palmitoyl Oligopeptide (and) Palmitoyl Tetrapeptide-7 | | | | | 3.00 |

| **Phase G** | | | | | | |
|---|---|---|---|---|---|---|
| Parfume | Fragrance | 0.10 | 0.10 | | | |

| | | | | | | |
|---|---|---|---|---|---|---|
| **Protocol:** Phase A: Sprinkle carbomer in water, let swell 15 minutes. Mix Phase B. Pour Phase B in Phase A, homogenise. Then weigh Phase C, mix and add to Phase A+B, under stirring. Let swell 1 hour. Extemporaneously add Phase D in the previous phase under stirring. Neutralize with Phase E. Put under stirring. Then add Phase F. Allow to mix at least 1 hour under stirring, and then add Phase G. Mix thoroughly. **Serum n°1 properties:** complexion lightening and face remodeling for more firmness and cohesion. **Serum n°2 properties:** association of the effect of the compound according to the invention with a protection effect against UV A and B radiations. **Serum n°3 properties:** association of the effect of the compound according to the invention with a hydration effect. **Serum n°4 properties:** association of the effect of the compound according to the invention with a regulation effect of seborreic production in case of oily skins. **Serum n°5 properties:** association of the effect of the compound according to the invention with an action against rings under the eyes and pro-inflammatory agents. | | | | | | |

### 3/5 Oil form

### Ingredient according to the invention:

Formulation comprising 2% w/w of the compound of the invention in a hydrophobic matrix

### Examples of other additional/optional ingredients:

REVIDRAT^{™} is an ingredient product marketed by SEDERMA, which in particular improve epidermal cohesion and its hydration.
RENOVAGE^{™} is an ingredient product marketed by SEDERMA (FR 02885522, WO 2006/120646) which fight all signs of aging and stress.

| **OIL FORM** | | **Weight %** | | |
|---|---|---|---|---|
| **Ingredients** | **INCI name** | **Oil n°1** | **Oil n°2** | **Oil n°3** |
| **Phase A** | | | | |
| Crodamol GTCC | caprylic /capric triglycerides | Qsp100 | Qsp100 | Qsp100 |
| DC 245 | Cyclopentasiloxane | 20.00 | 20.00 | 20.00 |
| Escalol 507 | Ethylhexyl dimethyl PABA | 2.00 | 2.00 | 2.00 |
| Escalol 557 | Ethylhexyl Methoxycinnamate | 2.00 | 2.00 | 2.00 |
| Crodamol OS | Ethylhexyl stearate | 20.00 | 20.00 | 20.00 |
| Coviox T 70 | Tocopherol Acetate | 0.20 | 0.20 | 0.20 |

| **Phase B** | | | | |
|---|---|---|---|---|
| Formula comprising 2% w/w of the invention compound in | / | 3.00 | 3.00 | 3.00 |
| an hydrophobic matrix | | | | |
| REVIDRAT™ | | | 3.00 | |
| RENOVAGE™ | Caprylic/Capric Triglyceride-Teprenone | | | 3.00 |

| **Phase C** | | | | |
|---|---|---|---|---|
| Fragrance | Fragrance | 0.10 | 0.10 | 0.10 |

| | | | | |
|---|---|---|---|---|
| **Protocol:** Weigh and homogenize Phase A under stirring. Add Phase B in Phase A and homogenise. Add Phase C in Phase (A+B) and homogenise. **Oil n°1 properties:** Oleodensifying effect, protective against UV solar radiations. **Oil n°2 properties:** REVIDRAT^{™} reinforce the effects of the compound of the invention adding a hydration dimension. **Oil n°3 properties:** RENOVAGE^{™} reinforce the antioxydant effect of the compound of the invention in delaying senescence. | | | | |

## Claims

1. Polyterpenic compound corresponding to formula I below: 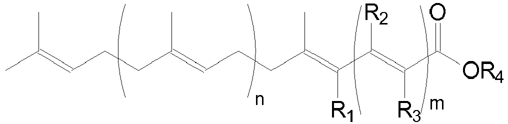
with n = 3, m = 0 or 1, R₁, R₂ and R₃, independently of each other a methyl or a hydrogen atom and R₄ is an ethyl or methyl group,
with the exclusion of the compounds corresponding in formula I to n=3, m=0, R₁=H and R₄=methyl or ethyl.

2. Compound (GGHE) according to claim 1, **characterized in that** it corresponds to the following formula II: 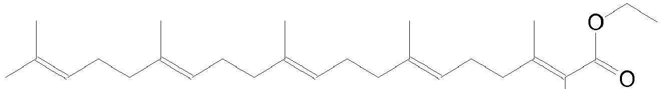 with n = 3, m = 0, R₁ = methyl and R₄ = ethyl.

3. Compound according to claim 1, **characterized in that** R₂ is a methyl.

4. Compound (GGOE) according to claim 3, **characterized in that** it corresponds to the following formula III: 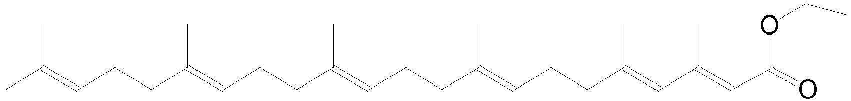 with n = 3, m = 1, R₁ = H, R₂ = methyl, R₃ = H and R₄ = ethyl.

5. Topical composition **characterized in that** it comprises, in a physiologically acceptable medium, as active ingredient, an effective amount of one or more compounds according to anyone of claims 1 to 4.

6. Composition according to claim 5, **characterized in that** it comprises at least one additional active ingredient selected from agents for lightening, anti-redness, sunscreening, moisturizing, humectants, exfoliating, anti-aging, anti-wrinkle and fine lines, stimulating the collagen and/or elastin synthesis, volumizing, elastic properties improving, anti-acne, anti-inflammatory, anti-oxidants, anti-free radical, depigmenting agents, depilatories, anti-regrowth or promoting the growth agents, peptides, vitamins.

7. Composition according to claim 5 or 6, **characterized in that** the amount of the active compound is comprised between 0.000001% and 15% w/w.

8. Composition according to claim 7, **characterized in that** the amount of the active compound is comprised between 0.0001% and 5% w/w.

9. Use of the compounds according to anyone of claims 1 to 4 or of a composition according to anyone of claim 5 to 8; or of a compound (GGPM) having the following formula V: 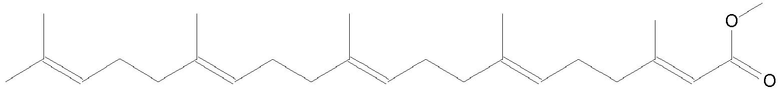 corresponding in formula I to n = 3, m = 0, R₁ = H and R₄ = methyl; or
of a compound (GGPE) having the following formula IV: 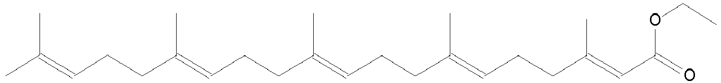 corresponding in fomula I to n = 3, m = 0, R₁ = H and R₄ = ethyl; or
a composition comprising as active ingredient an effective amount of the compound of formula V or the compound of formula IV in a physiologically acceptable medium,
for the cosmetic non therapeutic treatment of skin conditions to improve the appearance of the skin.

10. Use according to claim 9 for a topical treatment.

11. Use according to claim 9 or 10, for the prevention and treatment of skin aging.

12. Use according to anyone of claims 9 or 11, for an anti radical and/or anti oxidant treatment.

13. Use according to anyone of claims 9 to 12, for the treatment of loss of mechanical properties, in particular, skin tone, firmess and elasticity.

14. Use according to anyone of claims 9 to 13, for skin volumizing by stimulating the adipocyte synthesis.

15. Use according to anyone of claims 9 to 14, for skin lightening.

## Patentansprüche

1. Polyteipenverbindung, der nachfolgenden Formel I entsprechend: 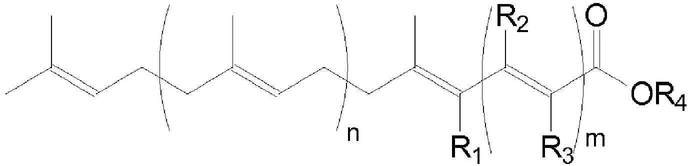 wobei n = 3, m = 0 oder 1, R₁, R₂ und R₃ jeweils unabhängig voneinander eine Methylgruppe oder ein Wasserstoffatom sind und R₄ eine Ethyl- oder Methylgruppe ist, wobei die Verbindungen ausgeschlossen sind, die in der Formel I n = 3, m = 0, R₁ = H und R₄= Methyl oder Ethyl entsprechen.

2. Verbindung (GGHE) nach Anspruch 1, **dadurch gekennzeichnet, dass** sie der nachfolgenden Formel II entspricht: 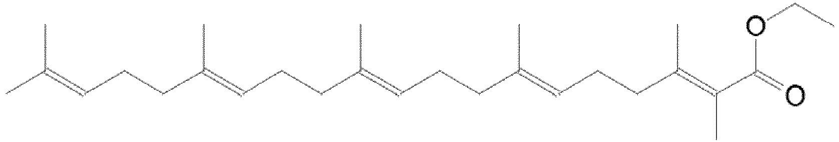 wobei n = 3, m = 0, R₁ = Methyl und R₄ = Ethyl.

3. Verbindung nach Anspruch 1, **dadurch gekennzeichnet, dass** R₂ eine Methylgruppe ist.

4. Verbindung (GGOE) nach Anspruch 3, **dadurch gekennzeichnet, dass** sie der nachfolgenden Formel III entspricht: 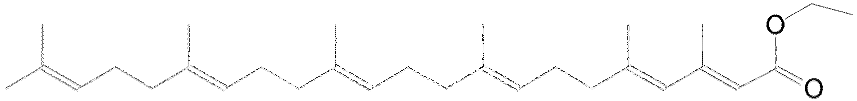 wobei n = 3, m = 1, R₁ = H, R₂ = Methyl, R₃ = H und R₄ = Ethyl.

5. Topische Zusammensetzung, **dadurch gekennzeichnet, dass** sie in einem physiologisch annehmbaren Medium als Wirkstoff eine wirksame Menge an mindestens einer Verbindung nach einem der Ansprüche 1 - 4 umfasst.

6. Zusammensetzung nach Anspruch 5, **dadurch gekennzeichnet, dass** sie mindestens einen weiteren Wirkstoff umfasst, der aus der nachfolgenden Gruppe gewählt ist: Hautaufhellungsmittel, Anti-Rötungsmittel, Sonnenschutzmittel, Feuchtigkeitsspender, Feuchthaltemittel, Exfoliationsmittel, Mittel gegen die Hautalterung, Mittel gegen (feine) Falten, Mittel zur Stimulation der Kollagen- und/oder Elastinsynthese, volumengebende Mittel, Mittel zur Verbesserung der elastischen Eigenschaften, Mittel gegen Akne, Antiinflammatoria, Mittel gegen freie Radikale, Depigmentierungsmittel, Enthaarungsmittel, Mittel gegen Neuwuchs oder zur Förderung von Neuwuchs, Peptide, Vitamine.

7. Zusammensetzung nach Anspruch 5 oder 6, **dadurch gekennzeichnet, dass** der Wirkstoffgehalt im Bereich zwischen 0,000001 und 15 % w/w liegt.

8. Zusammensetzung nach Anspruch 7, **dadurch gekennzeichnet, dass** der Wirkstoffgehalt im Bereich zwischen 0,0001 und 5 % w/w liegt.

9. Einsatz der Verbindungen nach einem der Ansprüche 1 - 4 oder einer Zusammensetzung nach einem der Ansprüche 5 - 8 oder einer Verbindung (GGPM) mit der nachfolgenden Formel V: 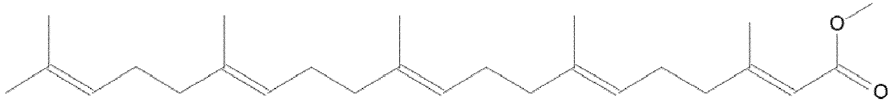 die in der Formel I n = 3, m = 0, R₁ = H und R₄ = Methyl entspricht; oder einer Verbindung (GGPE) der nachfolgenden Formel IV: 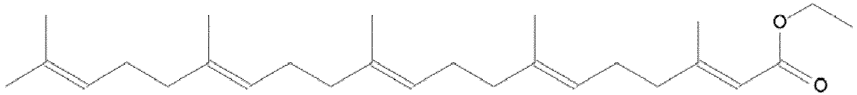 die in der Formel I n = 3, m = 0, R₁ = H und R₄ = Ethyl entspricht; oder
einer Zusammensetzung, die als Wirkstoff eine wirksame Menge an der Verbindung nach Formel V oder der Verbindung nach Formel IV in einem physiologisch annehmbaren Medium zur kosmetischen, nicht therapeutischen Behandlung von Hautzuständen zur Verbesserung des Erscheinungsbildes der Haut.

10. Einsatz nach Anspruch 9 zur topischen Behandlung.

11. Einsatz nach Anspruch 9 oder 10 zur Vorbeugung und Behandlung der Hautalterung.

12. Einsatz nach einem der Ansprüche 9 oder 11 zur Behandlung gegen freie Radikale die Oxidation.

13. Einsatz nach einem der Ansprüche 9 - 12 zur Behandlung des Verlustes mechanischer Eigenschaften, insbesondere von Hautton, Hautfestigkeit und Elastizität.

14. Einsatz nach einem der Ansprüche 9 - 13 zur Erhöhung der Hautfülle durch Stimulieren der Adipozytensynthese.

15. Einsatz nach einem der Ansprüche 9 - 14 zur Hautaufhellung.

## Revendications

1. Composé polyterpénique de formule développée I suivante : 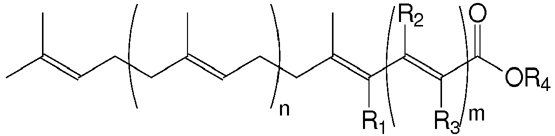
avec n = 3, m = 0 ou 1, R₁, R₂ et R₃, indépendamment les uns des autres, un méthyle ou un atome d'hydrogène et R₄ est un groupe éthyle ou méthyle,
à l'exclusion des composés correspondant dans la formule I à n = 3, m = 0, R ₁= H et R₄ = méthyle ou éthyle.

2. Composé (GGHE) selon la revendication 1, **caractérisé en ce qu'**il correspond à la formule II suivante : 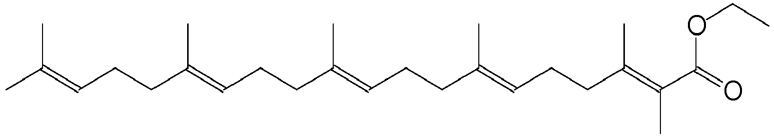 avec n = 3, m = 0, R₁ = méthyle et R₄= éthyle.

3. Composé selon la revendication 1, **caractérisé en ce que** R₂ est un méthyle.

4. Composé (GGOE) selon la revendication 3, **caractérisé en ce qu'**il correspond à la formule III suivante : 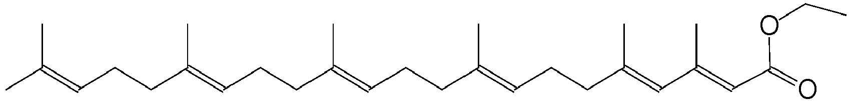 avec n = 3, m = 1, R₁ = H, R₂ = méthyle, R₃ = H et R₄ = éthyle.

5. Composition topique **caractérisée en ce qu'**elle comprend, dans un milieu physiologiquement acceptable, en tant qu'agent actif, une quantité efficace d'un ou plusieurs composés polyterpéniques selon l'une quelconque des revendications 1 à 4.

6. Composition selon la revendication 5, **caractérisée en ce qu'**elle comprend au moins un ingrédient actif additionnel choisi parmi les agents éclaircissants, anti-rougeurs, filtres solaires, hydratants, humectants, exfoliants, anti-âges, anti-rides et ridules, stimulant la synthèse de collagène et/ou d'élastine, volumateurs, améliorant les propriétés élastiques, anti-acné, anti-inflammatoires, antioxydants, anti-radicalaires, dépigmentants, dépilatoires, anti-repousse, ou favorisant la pousse, peptides, vitamines.

7. Composition selon la revendication 5 ou 6, **caractérisée en ce que** la quantité en composé actif est comprise entre 0.000001% et 15% p/p.

8. Composition selon la revendication 7, **caractérisée en ce que** la quantié en composé actif est comprise entre 0.0001% et 5% p/p.

9. Utilisation des composés selon l'une quelconque des revendications 1 à 4 ou d'une composition selon l'une quelconque des revendications 5 à 8 ; ou d'un composé (GGPM) ayant la formule V suivante : 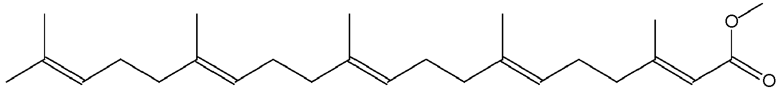 correspondant dans la formule I à n = 3, m = 0, R₁ = H et R₄ = méthyle ; ou d'un composé (GGPE) ayant la formule IV suivante : 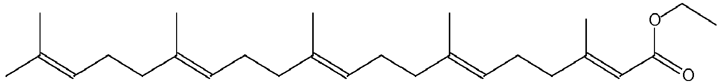 correspondant dans la formule I à n = 3, m = 0, R₁ = H et R₄ = éthyle ; ou
d'une composition comprenant comme ingrédient actif une quantité efficace du composé de formule V ou du composé de formule IV dans un milieu physiologiquement acceptable,
pour le traitement cosmétique non thérapeutique des états de la peau pour améliorer l'apparence de la peau.

10. Utilisation selon la revendication 9 pour un traitement topique.

11. Utilisation selon la revendication 9 ou 10, pour la prévention et le traitement des signes du vieillissement cutané.

12. Utilisation selon l'une quelconque des revendications 9 à 11, pour un traitement antiradicalaire et/ou antioxydant.

13. Utilisation selon l'une quelconque des revendications 9 à 12, pour le traitement de la perte de propriétés mécaniques, en particulier la tonicité, la fermeté et l'élasticité.

14. Utilisation selon l'une quelconque des revendications 9 à 13, pour donner du volume à la peau en stimulant la synthèse d'adipocytes.

15. Utilisation selon l'une quelconque des revendications 9 à 14, pour éclaircir la peau.


### SEQUENCE LISTING

<110> SEDERMA
<120> NEW POLYTERPENE TYPE COMPOUNDS, COMPOSITIONS CONTAINING THEM AND TOPICAL USES THEREOF
<130> GGder PCT
<150> FR1052672
   <151> 2010-04-08
<160> 11
<170> PatentIn version 3.5
<210> 1
   <211> 6
   <212> PRT
   <213> Artificial
<220>
   <223> Synthetic peptide
<220>
   <221> MOD_RES
   <222> (1)..(1)
   <223> AMIDATION, Pal (C16). Seq description Pal-Val-Gly-Val-Ala-Pro-Gly.
<400> 1
   Val Gly Val Ala Pro Gly
   1               5
<210> 2
   <211> 5
   <212> PRT
   <213> Artificial
<220>
   <223> Synthetic peptide
<220>
   <221> MOD_RES
   <222> (1)..(1)
   <223> AMIDATION, Pal (C16). Seq description Pal-Lys-Thr-Thr-Lys-Ser
<400> 2
   Lys Thr Thr Lys Ser
   1               5
<210> 3
   <211> 4
   <212> PRT
   <213> Artificial
<220>
   <223> Synthetic peptide
<220>
   <221> MOD_RES
   <222> (1)..(1)
   <223> AMIDATION, Pal (C16). Seq description Pal-Gly-Gln-Pro-Arg
<400> 3
   Gly Gln Pro Arg
   1
<210> 4
   <211> 5
   <212> PRT
   <213> Artificial
<220>
   <223> Synthetic peptide
<400> 4
   Lys Thr Thr Lys Ser
   1 5
<210> 5
   <211> 4
   <212> PRT
   <213> Artificial
<220>
   <223> synthetic peptide
<400> 5
   Arg Ser Arg Lys
   1
<210> 6
   <211> 4
   <212> PRT
   <213> Artificial
<220>
   <223> synthetic peptide
<400> 6
   Gly Gln Pro Arg
   1
<210> 7
   <211> 4
   <212> PRT
   <213> Artificial
<220>
   <223> synthetic peptide
<400> 7
   Lys Thr Phe Lys
   1
<210> 8
   <211> 6
   <212> PRT
   <213> Artificial
<220>
   <223> synthetic peptide
<400> 8
   Gly Lys Thr Thr Lys Ser
   1               5
<210> 9
   <211> 6
   <212> PRT
   <213> Artificial
<220>
   <223> Synthetic peptide
<400> 9
   Val Gly Val Ala Pro Gly
   1               5
<210> 10
   <211> 5
   <212> PRT
   <213> Artificial
<220>
   <223> Synthetic peptide
<220>
   <221> MOD_RES
   <222> (1)..(1)
   <223> AMIDATION, Pal (C16). Seq description Pal-Tyr-Gly-Gly-Phe-Met
<400> 10
   Tyr Gly Gly Phe Met
   1               5
<210> 11
   <211> 5
   <212> PRT
   <213> artificial
<220>
   <223> synthetic peptide
<220>
   <221> MOD_RES
   <222> (1)..(1)
   <223> AMIDATION, Pal (C16). Seq description Pal-Tyr-Gly-Gly-Phe-Met
<220>
   <221> MOD_RES
   <222> (1)..(1)
   <223> AMIDATION, Pal (C16). Seq description Pal-Tyr-Gly-Gly-Phe-Leu
<400> 11
   Tyr Gly Gly Phe Leu
   1               5